# EUROPEAN PATENT SPECIFICATION

(11) **EP 0 250 662 B1**
(45) Date of publication and mention of the grant of the patent: **24.08.1994**
(21) Application number: 86304919.3
(22) Date of filing: 25.06.1986
(51) Int. Cl.: C12Q 1/68, C12Q 1/04, C07H 21/00

(54) **Detection of mycoplasma by DNA hybridization**
Nachweis von Mykoplasma durch Hybridisierung von DNS
Détection de mycoplasma par hybridation d'ADN

(43) Date of publication of application: 07.01.1988
(73) Proprietor: THE REGENTS OF THE UNIVERSITY OF CALIFORNIA, Berkeley, California 94720 (US)
(72) Inventor: Gobel, Ulf, D-7800 Freiberg (DE); Stanbridge, Eric J., Corona Del Mar, CA 92625 (US)
(74) Representative: Newstead, Michael John

(56) References cited:
- EP-A- 0 076 123
- EP-A- 0 079 139
- EP-A- 0 120 658
- EP-A- 0 155 359
- EP-A- 0 155 360
- WO-A-84/01174
- WO-A-84/02721
- SCIENCE, vol. 209, 20 July 1980, London (GB); G.E. FOX et al., pp. 457-463#

## Description

### Field of the Invention

This invention relates generally to the field of Biology, and more particularly to the fields of Biomedicine, Biochemistry and Molecular Biology.

### Background and Summary of the Invention

Mycoplasmas are a group of pathogenic microorganisms of the Class Mollicutes characterized by having a small size and lacking a cell wall. These microorganisms are among the smallest-known organisms capable of a free living existence, and are important pathogens in man, plants and animals. For example, atypical pneumonia and non-gonococcal urethritis are common mycoplasma infections in man. Mycoplasmas have also been associated with rheumatoid arthritis, spontaneous abortion, infertility and other genital tract diseases, and certain autoimmune disease states. Moreover, mycoplasmas are common contaminants in cell cultures. In biological research, mycoplasma contamination of tissue culture is a serious problem which demands constant monitoring.

Not surprisingly, these organisms are extremely fastidious and at present there are no cost-effective specific diagnostic procedures to determine the presence of mycoplasma infections. The most commonly-employed detection methods for mycoplasmas in clinical samples are serological and cultural. The serological methods are subject to false positives and the cultural methods are costly, time consuming and tedious. Many of the biochemical techniques in current usage for detection of microbial contaminants in cell cultures do not specifically detect mycoplasmas but rather indicate the presence of any prokaryote or simple eukaryote such as yeast and fungi, and some may even detect viruses. Such a test is advantageous if one is interested only in the knowledge that a microbial agent is present, but if one is searching for a suspected etiological agent of an animal or human disease it is obviously necessary to classify the agent as fully as possible.

Further, the above procedures are hampered by special problems. For example, there are apparently "non-cultivable" mycoplasmas which are not detected by conventional culture methods. In addition, in the case of immunofluorescence tests more than one antibody might be required to identify the particular organism since more than nine different mycoplasma species are common tissue culture contaminants. Also, DNA stains are not necessarily mycoplasma-specific.

Therefore, a simple, sensitive, specific, cost-effective, and rapid mycoplasma detection system has been a desideratum in the fields of diagnostic medicine and biological research.

The use of nucleotide sequence homology and nucleic acid hybridization kinetics has become a widely-employed technique for detecting various organisms in cells and cell cultures. However, prior to this invention reliable and specific DNA probes have not been available for mycoplasma detection.

WO 84/02721 describes a system for detecting the presence of Legionella genus organisms and bacteria in general. However, there is no teaching or suggestion as to how the specificities may be improved, or of particular probe material.

The present invention proceeds by the use of specific mycoplasma ribosomal RNA gene fragments which are labeled or tagged by a variety of techniques, such as radioisotope labeling, biotin labeling, PEI-peroxidase conjugates, or fluorescent antibody tagging ELISA methods, for the specific and sensitive detection of mycoplasmas in clinical specimens, cells or cell cultures by DNA or RNA hybridization.

In one aspect of the invention, a DNA sequence from the 16S RNA gene of mycoplasma is provided, which includes a nucleotide sequence selected from the group consisting of AACACGTATC, CGAATCAGCTATGTCG, GAGGTT―AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, and TCTCGGGTCT, which code for mycoplasma ribosomal RNA (rRNA) where T represents thymine, G represent guanine, A represents adenine, C represents cytosine and - indicates a nucleotide deletion within the sequence with respect to the comparable sequence in E. coli. These fragments differ significantly from the 16S RNA gene of E. coli, and thus form the basis for mycoplasma-specific probes which are constituted of labeled nucleotide sequences complementary to the above.

In another aspect of the invention, identified DNA sequences of a 16S RNA gene are provided which include nucleotide sequences selected from the group consisting of ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, and TGGATCACCTCCTT, which code for prokaryotic rRNA. These fragments represent regions within the 16S RNA gene that are identical for E. coli and all mycoplasmas examined. Universal probes for all prokaryotes are constituted of labeled nucleotide sequences complementary to these fragments.

The invention provides a method for the identification of a particular nucleotide sequence for use as a mycoplasma-specific probe or a probe specific for Mollicutes in general, which comprises:
a. cloning the entire operon of ribosomal RNA or portions thereof, of a particular species of mycoplasma into a bacteriophage or plasmid vector;
b. probing the resulting ribosomal RNA gene fragments with a non-mycoplasma prokaryotic ribosomal RNA operon or fragments thereof;
c. characterizing the fragments which hybridize with said non-mycoplasma prokaryotic ribosomal RNA operon;
d. identifying mycoplasma-specific fragments by differential hybridization;
e. subcloning mycoplasma-specific fragments into a sequencing vector;
f. sequencing the resulting subcloned mycoplasma-specific fragments;
g. repeating steps a, b, c, d, e, and f for other members of the - Class Mollicutes;
h. comparing sequences obtained in steps f and g; and
i. identifying a sequence common to all the species of mycoplasma but differing from the corresponding sequence in non-mycoplasmal prokaryotes for use as a mycoplasma-specific probe; or sequences specific for a given mycoplasma, ureaplasma, acholeplasma, or spiroplasma species for use as probes specific for individual species of mycoplasmas, ureaplasmas, acholeplasmas and spiroplasmas.

This method may be used in a method for determining the presence of a prokaryotic organism of the Class Mollicutes which contains a nucleic acid including a particular nucleotide sequence which is present in nucleic acids from said prokaryotic organisms but absent in nucleic acids from eukaryotic organisms, which comprises:
identifying the particular nucleotide sequence according to the identification method described above;
contacting a medium which may contain a nucleic acid or nucleic acid fragment from said prokaryotic organism including said particular nucleotide sequence with an oligonucleotide including a nucleotide sequence complementary to said particular nucleotide sequence;
whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide.

Other aspects of the invention concern the specific biological probes used for detecting mycoplasmas or prokaryotes in general in accordance with the above described process and the identification and production of such probes.

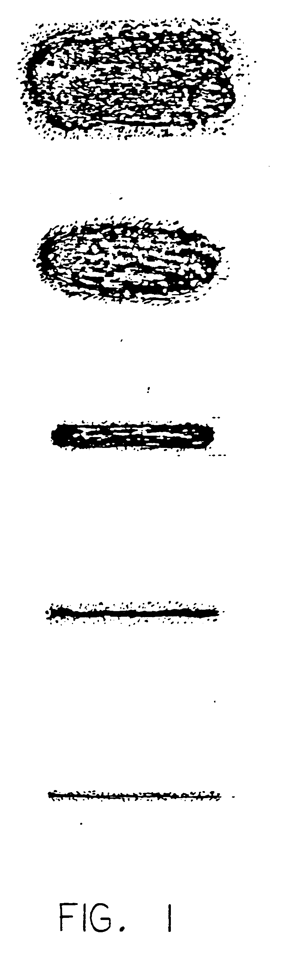

### BRIEF DESCRIPTION OF THE DRAWING

The sole figure of the drawing shows a slot blot development illustrating the present invention.

### DESCRIPTION OF THE PREFERRED EMBODIMENTS

The invention will now be described by detailing first the specific steps involved in producing and using the biological probes of the present invention and then describing how particular nucleotide sequences useful in this invention are determined.

### SYNTHESIS OF DEOXYOLIGONUCLEOTIDES

The 16 bp deoxyoligonucleotide, GCTTAGTCGATACAGC, which is complementary to one of the 16S mycoplasma RNA gene sequences listed above, was synthesized using the phosphotriester solid phase procedure described in M. H. Caruthers et al., Genetic Engineering, Vol. 4, p. 1-17, Plenum Publishing Co. (1982), which is hereby incorporated by reference.

Any of the other deoxyoligonucleotides previously mentioned or any other desired oligonucleotide can be similarly prepared. For example, instead of a DNA probe it may be desired to synthesize an RNA probe such as a recombinant SP6 vector transcript containing the sequence CGAAUCAGCUAUGUCG.

DNA-RN A or RNA-RNA hybridization to ribosomal RNA molecules amplifies the sensitivity of the detection several hundredfold above any DNA-DNA or RNA-DNA hybridization using probes against genomic DNA sequences, since the use of such probe which will detect multiple copies of ribosomal RNA per mycoplasma or eubacterial cell.

### TESTING OF DEOXYOLIGONUCLEOTIDES

The above described deoxyoligonucleotide was ³²P-labeled at the 5' end using the procedure in C. C. Richardson, Procedures in Nucleic Acid Research (Cantoni, G. L. and Davies, D. R., eds.), Vol. 2, pp. 815-828, Harper and Row, New York (1971), which is hereby incorporated by reference. The resulting labeled deoxyoligonucleotide was then used as a mycoplasma-specific probe. Specificity for mycoplasma was demonstrated by means of slot blot hybridization as described in M. Cunningham, Anal. Biochem. 128:415-421 (1983), which is herein incorporated by reference. For this purpose a 1600 bp DNA fragment of Mycoplasma pneumoniae which had been cloned into pUC8 was used. The 1600 bp fragment contains the above described 16 base deoxyoligonucleotide. A genomic digest of E. coli, a representative prokaryotic eubacterium, was also produced by digestion with the enzyme HindIII. The digested E. coli DNA and the 1600 bp M. pneumoniae DNA fragment were transferred onto nitrocellulose filters according to the procedure in the J. J. Leary et al., Proc. Natl. Acad. Sci. U.S.A. 80:4045-4049 (1983), which is hereby incorporated by reference. The nitrocellulose filters containing the DNA fragments were baked for 2 hours at 80°C under reduced pressure and hybridized to the ³²P-labeled deoxyoligonucleotide. Development of the resulting slot blots, shown in the drawing, revealed blots of increasing intensity for the M. pneumoniae DNA segment at 0.00034 ng, 0.0034 ng, 0.034 ng, 0.34 ng, and 3.4 ng (calculated for 16 bp) and no blots for the E. coli DNA segment at 0.00015 »g, 0.0015 »g, 0.015 »g, 0.15 »g,and 1.5 »g indicating the specificity of the deoxyoligonucleotide probe for mycoplasma. The deoxyoligonucleotide having the sequence GCTTAGTCGATACAGC thus is useful as a mycoplasma-specific probe which hybridizes with mycoplasmal DNA but does not hybridize with DNA of other prokaryotic organisms. On the other hand, a deoxyotigonucleotide having the sequence TGCCCACTCA, for example, which is complementary to one of the prokaryotic coding gene sequences, is useful as a prokaryote-speciflc probe which hybridizes with prokaryotes but not with eukaryotes.

For the detection of mycoplasmas in infected cells the following procedure has been found effective. The cells are trypsinized using 1-2 T75 tissue culture flasks with Trypsin EDTA (0.05% trypsin, 0.04% EDTA in PBS) for 2 minutes at 37°C. The trypsinized cells are resuspended in 1-2 ml of growth medium and spotted in a quantity of 50-100 »l (1 x 10⁵-1 x 10⁶ cells) onto a nitrocellulose filter wetted with 10 x SSC (1 x SSC: 15 mM Na citrate, 150 mM NaCl, pH 7.4) using a Minifold II slot blot hybridization apparatus available from Schleicher and Schuell, Inc., Keene, N. H. The DNA samples applied to the slot blots are denatured with alkali (0.5 M NaOH, 1.5 M NaCl) for 5-10 minutes at room temperature and neutralized for 5-10 minutes at room temperature using 0.5 M Tris, pH 7.2 and 3.0 M NaCl. The filter is then washed with 2 x SSC for five minutes at room temperature and baked in a vacuum oven for 2 hours at 80°C. The filter is prehybridized for 2 hours at 65°C using a prehybridization buffer consisting of 0.5 mM EDTA, 5 mM Tris, pH 7.5, 5 x Denhardt, and 100 »g/ml heat denatured herring sperm DNA. Hybridization, using the probes of this invention in a concentration measured as 1-2 x 10⁶ cpm of ³²P-labeled deoxyoligonucleotide, specific activity >10⁸ cpm/»g in hybridization buffer consisting of 10 mM Tris, pH 7.5, 1 mM EDTA, 0.75 M NaCl, 1 x Denhardt, 0.5% SDS, 10% dextran sulfate and 100 »g/ml heat denatured herring sperm DNA, is carried out at 65°C for 16 hours. Following hybridization the filter is washed 2-4 hours at 65°C with 2 x SET, 0.2% SDS (1 x SET:30 mM Tris, pH 8.0, 150 mM NaCl) and 1-2 hours at room temperature with 4 mM Tris base. The filter is then dried and exposed on X-ray film using 1 or 2 Dupont Cronex intensifying screens.

### DETERMINATION OF PARTICULAR NUCLEOTIDE SEQUENCES

While the foregoing description of the present invention teaches how particular nucleotide sequences can be prepared and used, the broader scope of this invention may be realized by examining the techniques used for determining particular nucleotide sequences which are useful as mycoplasma-specific probes, probes specific for prokaryotes in general, probes specific for individual mycoplasma, ureaplasma, acholeplasma, and spiroplasma species.

Cloning of the ribosomal RNA genome of M. pneumoniae was accomplished by HindIII digestion of total M. pneumoniae DNA and ligation of the HindIII fragments to the HindIII digested vector pUC8. The resulting ribosomal RNA gene fragments were probed with E. coli ribosomal RNA operon in the pKK 3535 plasmid according to the procedure in Gobel et al., Science, 226:1211-1213 (1984), which is hereby incorporated by reference, to identify cloned fragments which contained ribosomal sequences. A 1600 bp fragment was chosen on the basis of hybridization to mycoplasma species and not to E. coli or mammalian DNA under stringent hybridization conditions. This 1600 bp fragment was removed from the pUC8 vector by means of HindIII digestion and ligated to M13Mp8 DNA bacterial virus for sequencing using the Sanger dideoxy method described in Sanger et al., Proc. Natl. Acad. Sci. U.S.A., 74:5463-5467 (1977), which is hereby incorporated by reference.

Comparison of the sequences of the mycoplasma species M. pneumoniae, M. capricolum, and Mycoplasma species PG50 with E. coli indicated that certain sequences were common to all these species of mycoplasma but different from E. coli. These sequences could be synthesized and labeled and used as mycoplasma-specific probes. For example, GCTTAGTCGATACAGC constitued a mycoplasma-specific probe. Other sequences were common to these species of mycoplasma as well as E. coli. These latter sequences could be synthesized, labeled, and used as probes specific for all prokaryotic species. Still other sequences were unique to a single mycoplasma species and could be synthesized, labeled, and used as mycoplasma species-specific probes.

The present invention thus provides a specific, sensitive, and rapid method for the detection of mycoplasmas in contaminated cell cultures or other biological environments. Alternatively, the present invention can be used to provide a ribosomal DNA probe derived from a domain conserved in all prokaryotes. Such a probe would be extremely useful in the rapid and sensitive diagnosis of a bacteremia or septicemia in man or animals. The present invention may also be used to provide ribosomal DNA probes that are specific for individual mycoplasma, acholeplasma, ureaplasma, spiroplasma, and eubacterial species, respectively. These probes will be of particular use for those organisms where little or no information exists on their genetic make-up.

Although the present invention has been described in detail by reference to certain specific examples of deoxyoligonucleotides and mycoplasma species, it should be apparent to one skilled in the art that various modifications are possible. It is intended that this invention include such modifications and that the invention be limited only in accordance with the claims appended hereto.

## Claims (Claims for the following Contracting State(s): BE, CH, DE, FR, GB, IT, LI, LU, NL, SE)

1. A method for the identification of a particular nucleotide sequence for use as a mycoplasma-specific probe or a probe specific for Mollicutes in general, which comprises:
a. cloning the entire operon of ribosomal RNA or portions thereof, of a particular species of mycoplasma into a bacteriophage or plasmid vector;
b. probing the resulting ribosomal RNA gene fragments with a non-mycoplasma prokaryotic ribosomal RNA operon or fragments thereof;
c. characterizing the fragments which hybridize with said non-mycoplasma prokaryotic ribosomal RNA operon;
d. identifying mycoplasma-specific fragments by differential hybridization;
e. subcloning mycoplasma-speciflc fragments into a sequencing vector;
f. sequencing the resulting subcloned mycoplasma-specific fragments;
g. repeating steps a, b, c, d, e, and f for other members of the - Class Mollicutes;
h. comparing sequences obtained in steps f and g; and
i. identifying a sequence common to all the species of mycoplasma but differing from the corresponding sequence in non-mycoplasmal prokaryotes for use as a mycoplasma-specific probe; or sequences specific for a given mycoplasma, ureaplasma, acholeplasma, or spiroplasma species for use as probes specific for individual species of mycoplasmas, ureaplasmas, acholeplasmas and spiroplasmas.

2. A method for determining the presence of a prokaryotic organism of the Class Mollicutes which contains a nucleic acid including a particular nucleotide sequence which is present in nucleic acids from said prokaryotic organisms but absent in nucleic acids from eukaryotic organisms, which comprises:
identifying the particular nucleotide sequence according to the method of claim 1;
contacting a medium which may contain a nucleic acid or nucleic acid fragment from said prokaryotic organism including said particular nucleotide sequence with an oligonucleotide including a nucleotide sequence complementary to said particular nucleotide sequence;
whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide.

3. A method according to Claim 1 or 2 wherein the prokaryotic organism is a species of the genus Mycoplasma and said particular nucleotide sequence is present only in prokaryotic nucleic acids from species of the genus Mycoplasma.

4. A method according to Claim 1 or 2 wherein the prokaryotic organism is Mycoplasma capricolum and said particular nucleotide sequence is present only in prokaryotic nucleic acids from Mycoplasma capricolum.

5. A method according to Claim 1 or 2 wherein the prokaryotic organism is Mycoplasma species PG50 and said particular nucleotide sequence is present only in prokaryotic nucleic acids from Mycoplasma species PG50.

6. A method according to Claim 1 or 2 wherein the prokaryotic organism is Mycoplasma pneumoniae and said particular nucleotide sequence is present only in prokaryotic nucleic acids from Mycoplasma pneumoniae.

7. A method according to Claim 1 or 2 wherein the prokaryotic organism is a ureaplasma species and said particular nucleotide sequence is present only in prokaryotic nucleic acids from that said ureaplasma species.

8. A method according to Claim 1 or 2 wherein the prokaryotic organism is a spiroplasma species and said particular nucleotide sequence is present only in prokaryotic nucleic acids from that said, spiroplasma species.

9. A method according to Claim 1 or 2 wherein the prokaryotic organism is a acholeplasma species and said particular nucleotide sequence is present only in prokaryotic nucleic acids from that said acholeplasma species.

10. A method according to Claim 1 or 2 wherein said nucleic acid fragment including said particular nucleotide sequence is the 16S RNA gene.

11. A method according to Claim 1 or 2 wherein said nucleic acid fragment including said particular nucleotide sequence is the 23S RNA gene.

12. A method according to Claim 1 or 2 wherein said nucleic acid fragment including said particular nucleotide sequence is the 5S RNA gene.

13. A method for determining the presence of a prokaryotic organism of the class Mollicutes which contains a nucleic acid including a particular nucleotide sequence which is present in nucleic acids from said prokaryotic organisms but absent in nucleic acids from eukaryotic organisms, which comprises:
contacting a medium which may contain a nucleic acid or nucleic acid fragment from said prokaryotic organism including said particular nucleotide sequence with an oligonucleotide including a nucleotide sequence complementary to said particular nucleotide sequence, whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide; characterized in that said particular nucleotide sequence includes, at least one of the following mycoplasma-specific sequences or a sequence complementary to at least one of the following sequences: AACACGTATC, CGAATCAGCTATGTCG,
GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT and TAGATATATG.

14. A method for determining the presence of a prokaryotic organism which includes a nucleic acid including a particular nucleotide sequence which is present in nucleic acids from prokaryotic organisms but absent in nucleic acids from eukaryotic organisms, which comprises:
contacting a medium which may contain a nucleic acid or nucleic acid fragment from said prokaryotic organism including said particular nucleotide sequence with an oligonucleotide including a nucleotide sequence complementary to said particular nucleotide sequence, whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide;
characterized in that said particular nucleotide sequence includes at least one of the following prokaryotic sequences or a sequence complementary to at least one of the following sequences: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA and TGGATCACCTCCTT.

15. A mycoplasma-specific probe, characterized in that it hybridizes with a particular nucleotide sequence including at least one of the following mycoplasma-specific sequences or a sequence complementary to at least one of the following sequences: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT and TAGATATATG.

16. A prokaryote-specific probe, characterized in that it hybridizes with a particular nucleotide sequence including at least one of the following prokaryotic sequences or a sequence complementary to at least one of the following sequences: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, CGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA and TGGATCACCTCCTT.

17. An oligonucleotide containing a nucleotide sequence selected from the group consisting of AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG; TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, TGGATCACCTCCTT, and sequences complementary thereto, wherein T represents thymine, G represents guanine, A represents adenine, C represents cytosine and - indicates a nucleotide deletion within the sequence.

18. A deoxyoligonucleotide containing the nucleotide sequence GCTTAGTCGATACAGC.

19. An oligonucleotide containing the nucleotide sequence CGAAUCAGCUAUGUCG.

## Claims (Claims for the following Contracting State(s): AT)

1. A method for the identification of a particular nucleotide sequence for use as a mycoplasma-specific probe or a probe specific for Mollicutes in general, which comprises:
a. cloning the entire operon of ribosomal RNA or portions thereof, of a particular species of mycoplasma into a bacteriophage or plasmid vector;
b. probing the resulting ribosomal RNA gene fragments with a non-mycoplasma prokaryotic ribosomal RNA operon or fragments thereof;
c. characterizing the fragments which hybridize with said non-mycoplasma prokaryotic ribosomal RNA operon;
d. identifying mycoplasma-specific fragments by differential hybridization;
e. subcloning mycoplasma-specific fragments into a sequencing vector;
f. sequencing the resulting subcloned mycoplasma-specific fragments;
g. repeating steps a, b, c, d, e and f for other members of the Class Mollicutes;
h. comparing sequences obtained in steps f and g; and
i. identifying a sequence common to all the species of mycoplasma but differing from the corresponding sequence in non-mycoplasmal prokaryotes for use as a mycoplasma specific probe; or sequences specific for a given mycoplasma, ureaplasma, acholeplasma, or spiroplasma species for use as probes specific for individual species of mycoplasmas, ureaplasmas, acholeplasmas and spiroplasmas.

2. A method for determining the presence of a prokaryotic organism of the Class Mollicutes which contains a nucleic acid including a particular nucleotide sequence which is present in nucleic acids from said prokaryotic organisms but absent in nucleic acids from eukaryotic organisms, which comprises:
identifying the particular nucleotide sequence according to the method of claim 1;
contacting a medium which may contain a nucleic acid or nucleic acid fragment from said prokaryotic organism including said particular nucleotide sequence with an oligonucleotide including a nucleotide sequence complementary to said particular nucleotide sequence;
whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide.

3. A method according to claim 1 or 2 wherein the prokaryotic organism is a species of the genus Mycoplasma and said particular nucleotide sequence is present only in prokaryotic nucleic acids from species of the genus Mycoplasma.

4. A method according to claim 1 or 2 wherein the prokaryotic organism is Mycoplasma capricolum and said particular nucleotide sequence is present only in prokaryotic nucleic acids from Mycoplasma capricolum.

5. A method according to claim 1 or 2 wherein the prokaryotic organism is Mycoplasma species PG50 and said particular nucleotide sequence is present only in prokaryotic nucleic acids from Mycoplasma species PG50.

6. A method according to claim 1 or 2 wherein the prokaryotic organism is Mycoplasma pneumoniae and said particular nucleotide sequence is present only in prokaryotic nucleic acids from Mycoplasma pneumoniae.

7. A method according to claim 1 or 2 wherein the prokaryotic organism is a ureaplasma species and said particular nucleotide sequence is present only in prokaryotic nucleic acids from that said ureaplasma species.

8. A method according to claim 1 or 2 wherein the prokaryotic organism is a spiroplasma species and said particular nucleotide sequence is present only in prokaryotic nucleic acids from that said spiroplasma species.

9. A method according to claim 1 or 2 wherein the prokaryotic organism is a acholeplasma species and said particular nucleotide sequence is present only in prokaryotic nucleic acids from that said acholeplasma species.

10. A method according to claim 1 or 2 wherein said nucleic acid fragment including said particular nucleotide sequence is the 16S RNA gene.

11. A method according to claim 1 or 2 wherein said nucleic acid fragment including said particular nucleotide sequence is the 23S RNA gene.

12. A method according to claim 1 or 2 wherein said nucleic acid fragment including said particular nucleotide sequence is the 5S RNA gene.

13. A method for determining the presence of a prokaryotic organism of the class Mollicutes which contains a nucleic acid including a particular nucleotide sequence which is present in nucleic acids from said prokaryotic organisms but absent in nucleic acids from eukaryotic organisms, which comprises:
contacting a medium which may contain a nucleic acid or nucleic acid fragment from said prokaryotic organism including said particular nucleotide sequence with an oligonucleotide including a nucleotide sequence complementary to said particular nucleotide sequence, whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide;
characterized in that said particular nucleotide sequence includes at least one of the following mycoplasma-specific sequences or a sequence complementary to at least one of the following sequences: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT and TAGATATATG.

14. A method for determining the presence of a prokaryotic organism which includes a nucleic acid including a particular nucleotide sequence which is present in nucleic acids from prokaryotic organisms but absent in nucleic acids from eukaryotic organisms, which comprises:
contacting a medium which may contain a nucleic acid or nucleic acid fragment from said prokaryotic organism including said particular nucleotide sequence with an oligonucleotide including a nucleotide sequence complementary to said particular nucleotide sequence, whereby said oligonucleotide hybridizes with any nucleic acid or nucleic acid fragment from said prokaryotic organism including said particular nucleotide sequence which may be present in said medium; and
detecting the presence of any nucleic acid or nucleic acid fragment hybridized with said oligonucleotide;
characterized in that said particular nucleotide sequence includes at least one of the following prokaryotic sequences or a sequence complementary to at least one of the following sequences: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA and TGGATCACCTCCTT.

15. A method for preparing a mycoplasma-specific probe, characterized in that it hybridizes with a particular nucleotide sequence including at least one of the following mycoplasma-specific sequences or a sequence complementary to at least one of the following sequences: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT and TAGATATATG, which method comprises synthesizing in conventional manner a nucleotide sequence characteristic of the aforesaid probe.

16. A method for preparing a prokaryote-specific probe, characterized in that it hybridizes with a particular nucleotide sequence including at least one of the following prokaryotic sequences or a sequence complementary to at least one of the following sequences: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA and TGGATCACCTCCTT, which method comprises synthesizing in conventional manner a nucleotide sequence characteristic of the aforesaid probe.

17. A method for preparing an oligonucleotide containing a nucleotide sequence selected from the group consisting of AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, TGGATCACCTCCTT, and sequences complementary thereto, wherein T represents thymine, G represents guanine, A represents adenine, C represents cytosine and - indicates a nucleotide deletion within the sequence, which method comprises synthesizing in conventional manner a nucleotide sequence characteristic of the aforesaid oligonucleotide.

18. A method for preparing a deoxyoligonucleotide containing the nucleotide sequence GCTTAGTCGATACAGC, which method comprises synthesizing in conventional manner a nucleotide sequence characteristic of the aforesaid deoxyoligonucleotide.

19. A method for preparing an oligonucleotide containing the nucleotide sequence CGAAUCAGCUAUGUCG, which method comprises synthesizing in conventional manner a nucleotide sequence characteristic of the aforesaid oligonucleotide.

## Patentansprüche (Patentansprüche für folgende(n) Vertragsstaat(en): BE, CH, DE, FR, GB, IT, LI, LU, NL, SE)

1. Verfahren zur Identifizierung einer speziellen Nukleotidsequenz zur Verwendung als mykoplasmaspezifische Sonde oder als für Mollicutes im allgemeinen spezifische Sonde, wobei das Verfahren folgende Schritte aufweist:
a. Klonen des gesamten Operon von Ribosomen-RNA, oder Teilen davon, einer speziellen Spezies von Mykoplasma in einen bakteriophagen oder Plasmidvektor;
b. sondenmäßige Untersuchung der resultierenden Ribosomen-RNA-Genfragmente mit einem nicht-mykoplasmischen prokaryontischen Ribosomen-RNA-Operon oder Fragmenten davon;
c. Kennzeichnen der Fragmente, die mit dem nicht-mykoplasmischen prokaryontischen Ribosomen-RNA-Operon hybridisieren;
d. Identifizieren mykoplasmaspezififischer Fragmente durch differentielle Hybridisierung;
e. Unterklonen mykoplasmaspezifischer Fragmente in einen Sequenzierungsvektor;
f. Sequenzierung der resultierenden untergeklonten mykoplasmaspezifischen Fragmente;
g. Wiederholen der Schritte a, b, c, d, e und f für andere Mitglieder der Klasse Mollicutes;
h. Vergleichen der in Schritt f und Schritt g erhaltenen Sequenzen; und
i. Identifizieren einer Sequenz, die allen Mykoplasmaspezies gemeinsam ist, sich jedoch von der entsprechenden Sequenz in nicht-mykoplasmischen Prokaryonten zur Verwendung als mykoplasmaspezifische Sonde unterscheidet; oder von Sequenzen, die für eine bestimmte Mykoplasma-, Ureaplasma-, Acholeplasma- oder Spiroplasma-spezies spezifisch sind, zur Verwendung als Sonden, die für individuelle Spezies von Mykoplasmen, Ureaplasmen, Acholeplasmen und Spiroplasmen spezifisch sind.

2. Verfahren zum Bestimmen des Vorhandenseins eines prokaryontischen Organismus der Klasse Mollicutes, der eine Nukleinsäure mit einer speziellen Nukleotidsequenz enthält, die in Nukleinsäuren aus den prokaryontischen Organismen vorhanden ist, jedoch in Nukleinsäuren aus eukaryontischen Organismen nicht vorhanden ist,
wobei das Verfahren folgende Schritte aufweist:
Identifizieren der speziellen Nukleotidsequenz nach dem Verfahren von Anspruch 1;
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist;
wodurch das Oligonukleotid mit jeglicher Nukleinsäure oder jeglichem Nukleinsäurefragment aus dem die genannte spezielle Nukleotidsequenz enthaltenden prokaryontischen Organismus, die bzw. das in dem Medium vorhanden sein kann, hybridisiert; und
Detektieren des Vorhandenseins jeglicher Nukleinsäure oder jeglichen Nukleinsäurefragments, die bzw. das mit dem Oligonukleotid hybridisiert hat.

3. Verfahren nach Anspruch 1 oder 2,
wobei es sich bei dem prokaryontischen Organismus um eine Spezies der Gattung Mykoplasma handelt und die genannte spezielle Nukleotidsequenz nur in prokaryontischen Nukleinsäuren aus der Spezies der Gattung Mykoplasma vorhanden ist.

4. Verfahren nach Anspruch 1 oder 2,
wobei es sich bei dem prokaryontischen Organismums um Mykoplasma capricolum handelt und die genannte spezielle Nukleotidsequenz nur in prokaryontischen Nukleinsäuren aus Mykoplasma capricolum vorhanden ist.

5. Verfahren nach Anspruch 1 oder 2,
wobei es sich bei dem prokaryontischen Organismums um Mykoplasma der Spezies PG50 handelt und die genannte spezielle Nukleotidsequenz nur in prokaryontischen Nukleinsäuren aus Mykoplasma der Spezies PG50 vorhanden ist.

6. Verfahren nach Anspruch 1 oder 2,
wobei es sich bei dem prokaryontischen Organismums um Mykoplasma pneumoniae handelt und die genannte spezielle Nukleotidsequenz nur in prokaryontischen Nukleinsäuren aus Mykoplasma pneumoniae vorhanden ist.

7. Verfahren nach Anspruch 1 oder 2,
wobei es sich bei dem prokaryontischen Organismums um eine Ureaplasma-Spezies handelt und die genannte spezielle Nukleotidsequenz nur in prokaryontischen Nukleinsäuren aus dieser genannten Ureaplasma-Spezies vorhanden ist.

8. Verfahren nach Anspruch 1 oder 2,
wobei es sich bei dem prokaryontischen Organismums um eine Spiroplasma-Spezies handelt und die genannte spezielle Nukleotidsequenz nur in prokaryontischen Nukleinsäuren aus dieser genannten Spiroplasma-Spezies vorhanden ist.

9. Verfahren nach Anspruch 1 oder 2,
wobei es sich bei dem prokaryontischen Organismums um eine Acholeplasma-Spezies handelt und die genannte spezielle Nukleotidsequenz nur in prokaryontischen Nukleinsäuren aus dieser genannten Acholeplasma-Spezies vorhanden ist.

10. Verfahren nach Anspruch 1 oder 2,
wobei es sich bei dem Nukleinsäurefragment, das die genannte spezielle Nukleotidsequenz enthält, um das 16S-RNA-Gen handelt.

11. Verfahren nach Anspruch 1 oder 2,
wobei es sich bei dem Nukleinsäurefragment, das die genannte spezielle Nukleotidsequenz enthält, um das 23S-RNA-Gen handelt.

12. Verfahren nach Anspruch 1 oder 2,
wobei es sich bei dem Nukleinsäurefragment, das die genannte spezielle Nukleotidsequenz enthält, um das 5S-RNA-Gen handelt.

13. Verfahren zum Bestimmen des Vorhandenseins eines prokaryontischen Organismus der Klasse Mollicutes, der eine Nukleinsäure mit einer speziellen Nukleotidsequenz enthält, die in Nukleinsäuren aus den prokaryontischen Organismen vorhanden ist, jedoch in Nukleinsäuren aus eukaryontischen Organismen nicht vorhanden ist,
wobei das Verfahren folgende Schritte aufweist:
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist, wodurch das Oligonukleotid mit jeglicher Nukleinsäure oder jeglichem Nukleinsäurefragment aus dem die spezielle Nukleotidsequenz enthaltenden prokaryontischen Organismus, die bzw. das in dem Medium vorhanden sein kann, hybridisiert; und Detektieren des Vorhandenseins einer jeglichen Nukleinsäure oder eines jeglichen Nukleinsäurefragments, die bzw. das mit dem Oligonukleotid hybridisiert hat;
dadurch gekennzeichnet, daß die genannte spezielle Nukleotidsequenz wenigstens eine der folgenden mykoplasmaspezifischen Sequenzen oder eine zu wenigstens einer der nachfolgenden Sequenzen komplementäre Sequenz beinhaltet:
AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT und TAGATATATG.

14. Verfahren zum Bestimmen des Vorhandenseins eines prokaryontischen Organismus, der eine Nukleinsäure mit einer speziellen Nukleotidsequenz aufweist, die in Nukleinsäuren aus prokaryontischen Organismen vorhanden ist, jedoch in Nukleinsäuren aus eukaryontischen Organismen nicht vorhanden ist,
wobei das Verfahren folgende Schritte aufweist:
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist, wodurch das Oligonukleotid mit jeglicher Nukleinsäure oder jeglichem Nukleinsäurefragment aus dem die genannte spezielle Nukleotidsequenz enthaltenden prokaryontischen Organismus, die bzw. das in dem Medium vorhanden sein kann, hybridisiert; und
Detektieren des Vorhandenseins einer jeglichen Nukleinsäure oder eines jeglichen Nukleinsäurefragments, die bzw. das mit dem Oligonukleotid hybridisiert hat;
dadurch gekennzeichnet, daß die genannte spezielle Nukleotidsequenz wenigstens eine der folgenden prokaryontischen Sequenzen oder eine zu wenigstens einer der nachfolgenden Sequenzen komplementäre Sequenz beinhaltet: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA und TGGATCACCTCCTT.

15. Mykoplasmaspezifische Sonde,
dadurch gekennzeichnet, daß sie mit einer speziellen Nukleotidsequenz hybridisiert, die wenigstens eine der folgenden mykoplasmaspezifischen Sequenzen oder eine zu wenigstens einer der nachfolgenden Sequenzen komplementäre Sequenz beinhaltet: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT und TAGATATATG.

16. Prokaryontenspezifische Sonde,
dadurch gekennzeichnet, daß sie mit einer speziellen Nukleotidsequenz hybridisiert, die wenigstens eine der folgenden prokaryontischen Sequenzen oder eine zu wenigstens einer der nachfolgenden Sequenzen komplementäre Sequenz beinhaltet: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA und TGGATCACCTCCTT.

17. Oligonukleotid, das eine Nukleotidsequenz enthält, die ausgewählt ist aus der Gruppe bestehend aus: AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, TGGATCACCTCCTT, sowie dazu komplementären Sequenzen, wobei T Thymin, G Guanin, A Adenin, C Cytosin und - eine Nukleotidauslöschung innerhalb der Sequenz bedeuten.

18. Deoxyoligonukleotid, das die Nukleotidsequenz GCTTAGTCGATACAGC enthält.

19. Oligonukleotid, das die Nukleotidsequenz CGAAUCAGCUAUGUCG enthält.

## Patentansprüche (Patentansprüche für folgende(n) Vertragsstaat(en): AT)

1. Verfahren zur Identifizierung einer speziellen Nukleotidsequenz zur Verwendung als mykoplasmaspezifische Sonde oder als für Mollicutes im allgemeinen spezifische Sonde, wobei das Verfahren folgende Schritte aufweist:
a. Klonen des gesamten Operon von Ribosomen-RNA, oder Teilen davon, einer speziellen Spezies von Mykoplasma in einen bakteriophagen oder Plasmidvektor;
b. sondenmäßige Untersuchung der resultierenden Ribosomen-RNA-Genfragmente mit einem nicht-mykoplasmischen prokaryontischen Ribosomen-RNA-Operon oder Fragmenten davon;
c. Kennzeichnen der Fragmente, die mit dem nicht-mykoplasmischen prokaryontischen Ribosomen-RNA-Operon hybridisieren;
d. Identifizieren mykoplasmaspezififischer Fragmente durch differentielle Hybridisierung;
e. Unterklonen mykoplasmaspezifischer Fragmente in einen Sequenzierungsvektor;
f. Sequenzierung der resultierenden untergeklonten mykoplasmaspezifischen Fragmente;
g. Wiederholen der Schritte a, b, c, d, e und f für andere Mitglieder der Klasse Mollicutes;
h. Vergleichen der in Schritt f und Schritt g erhaltenen Sequenzen; und
i. Identifizieren einer Sequenz, die allen Mykoplasmaspezies gemeinsam ist, sich jedoch von der entsprechenden Sequenz in nicht-mykoplasmischen Prokaryonten zur Verwendung als mykoplasmaspezifische Sonde unterscheidet; oder von Sequenzen, die für eine bestimmte Mykoplasma-, Ureaplasma-, Acholeplasma- oder Spiroplasma-spezies spezifisch sind, zur Verwendung als Sonden, die für individuelle Spezies von Mykoplasmen, Ureaplasmen, Acholeplasmen und Spiroplasmen spezifisch sind.

2. Verfahren zum Bestimmen des Vorhandenseins eines prokaryontischen Organismus der Klasse Mollicutes, der eine Nukleinsäure mit einer speziellen Nukleotidsequenz enthält, die in Nukleinsäuren aus den prokaryontischen Organismen vorhanden ist, jedoch in Nukleinsäuren aus eukaryontischen Organismen nicht vorhanden ist,
wobei das Verfahren folgende Schritte aufweist:
Identifizieren der speziellen Nukleotidsequenz nach dem Verfahren von Anspruch 1;
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist;
wodurch das Oligonukleotid mit jeglicher Nukleinsäure oder jeglichem Nukleinsäurefragment aus dem die genannte spezielle Nukleotidsequenz enthaltenden prokaryontischen Organismus, die bzw. das in dem Medium vorhanden sein kann, hybridisiert; und
Detektieren des Vorhandenseins jeglicher Nukleinsäure oder jeglichen Nukleinsäurefragments, die bzw. das mit dem Oligonukleotid hybridisiert hat.

3. Verfahren nach Anspruch 1 oder 2,
wobei es sich bei dem prokaryontischen Organismus um eine Spezies der Gattung Mykoplasma handelt und die genannte spezielle Nukleotidsequenz nur in prokaryontischen Nukleinsäuren aus der Spezies der Gattung Mykoplasma vorhanden ist.

4. Verfahren nach Anspruch 1 oder 2,
wobei es sich bei dem Prokaryontischen Organismums um Mykoplasma capricolum handelt und die genannte spezielle Nukleotidsequenz nur in prokaryontischen Nukleinsäuren aus Mykoplasma capricolum vorhanden ist.

5. Verfahren nach Anspruch 1 oder 2,
wobei es sich bei dem prokaryontischen Organismums um Mykoplasma der Spezies PG50 handelt und die genannte spezielle Nukleotidsequenz nur in prokaryontischen Nukleinsäuren aus Mykoplasma der Spezies PG50 vorhanden ist.

6. Verfahren nach Anspruch 1 oder 2,
wobei es sich bei dem Prokaryontischen Organismums um Mykoplasma pneumoniae handelt und die genannte spezielle Nukleotidsequenz nur in prokaryontischen Nukleinsäuren aus Mykoplasma pneumoniae vorhanden ist.

7. Verfahren nach Anspruch 1 oder 2,
wobei es sich bei dem prokaryontischen Organismums um eine Ureaplasma-Spezies handelt und die genannte spezielle Nukleotidsequenz nur in prokaryontischen Nukleinsäuren aus dieser genannten Ureaplasma-Spezies vorhanden ist.

8. Verfahren nach Anspruch 1 oder 2,
wobei es sich bei dem prokaryontischen Organismums um eine Spiroplasma-Spezies handelt und die genannte spezielle Nukleotidsequenz nur in prokaryontischen Nukleinsäuren aus dieser genannten Spiroplasma-Spezies vorhanden ist.

9. Verfahren nach Anspruch 1 oder 2,
wobei es sich bei dem prokaryontischen Organismums um eine Acholeplasma-Spezies handelt und die genannte spezielle Nukleotidsequenz nur in prokaryontischen Nukleinsäuren aus dieser genannten Acholeplasma-Spezies vorhanden ist.

10. Verfahren nach Anspruch 1 oder 2,
wobei es sich bei dem Nukleinsäurefragment, das die genannte spezielle Nukleotidsequenz enthält, um das 16S-RNA-Gen handelt.

11. Verfahren nach Anspruch 1 oder 2,
wobei es sich bei dem Nukleinsäurefragment, das die genannte spezielle Nukleotidsequenz enthält, um das 23S-RNA-Gen handelt.

12. Verfahren nach Anspruch 1 oder 2,
wobei es sich bei dem Nukleinsäurefragment, das die genannte spezielle Nukleotidsequenz enthält, um das 5S-RNA-Gen handelt.

13. Verfahren zum Bestimmen des Vorhandenseins eines prokaryontischen Organismus der Klasse Mollicutes, der eine Nukleinsäure mit einer speziellen Nukleotidsequenz enthält, die in Nukleinsäuren aus den prokaryontischen Organismen vorhanden ist, jedoch in Nukleinsäuren aus eukaryontischen Organismen nicht vorhanden ist,
wobei das Verfahren folgende Schritte aufweist:
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist, wodurch das Oligonukleotid mit jeglicher Nukleinsäure oder jeglichem Nukleinsäurefragment aus dem die spezielle Nukleotidsequenz enthaltenden prokaryontischen Organismus, die bzw. das in dem Medium vorhanden sein kann, hybridisiert; und Detektieren des Vorhandenseins einer jeglichen Nukleinsäure oder eines jeglichen Nukleinsäurefragments, die bzw. das mit dem Oligonukleotid hybridisiert hat;
dadurch gekennzeichnet, daß die genannte spezielle Nukleotidsequenz wenigstens eine der folgenden mykoplasmaspezifischen Sequenzen oder eine zu wenigstens einer der nachfolgenden Sequenzen komplementäre Sequenz beinhaltet: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT und TAGATATATG.

14. Verfahen zum Bestimmen des Vorhandenseins eines prokaryontischen Organismus, der eine Nukleinsäure mit einer speziellen Nukleotidsequenz aufweist, die in Nukleinsäuren aus prokaryontischen Organismen vorhanden ist, jedoch in Nukleinsäuren aus eukaryontischen Organismen nicht vorhanden ist,
wobei das Verfahren folgende Schritte aufweist:
Kontaktieren eines Mediums, das eine Nukleinsäure oder ein Nukleinsäurefragment aus dem die genannte spezielle Nukleotidsequenz enthaltenden prokaryontischen Organismus enthalten kann, mit einem Oligonukleotid, das eine Nukleotidsequenz aufweist, die zu der genannten speziellen Nukleotidsequenz komplementär ist, wodurch das Oligonukleotid mit jeglicher Nukleinsäure oder jeglichem Nukleinsäurefragment aus dem die genannte spezielle Nukleotidsequenz enthaltenden prokaryontischen Organismus, die bzw. das in dem Medium vorhanden sein kann, hybridisiert; und
Detektieren des Vorhandenseins einer jeglichen Nukleinsäure oder eines jeglichen Nukleinsäurefragments, die bzw. das mit dem Oligonukleotid hybridisiert hat;
dadurch gekennzeichnet, daß die genannte spezielle Nukleotidsequenz wenigstens eine der folgenden prokaryontischen Sequenzen oder eine zu wenigstens einer der nachfolgenden Sequenzen komplementäre Sequenz beinhaltet: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA und TGGATCACCTCCTT.

15. Verfahren zum Bilden einer mykoplasmaspezifischen Sonde,
dadurch gekennzeichnet, daß sie mit einer speziellen Nukleotidsequenz hybridisiert, die wenigstens eine der folgenden mykoplasmaspezifischen Sequenzen oder eine zu wenigstens einer der nachfolgenden Sequenzen komplementäre Sequenz beinhaltet: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT und TAGATATATG, wobei das Verfahren eine in herkömmlicher Weise erfolgende Synthetisierung einer für die vorstehend gennante Sonde charakteristischen Nukleotidsequenz beinhaltet.

16. Verfahren zum Bilden einer prokaryontenspezifischen Sonde,
dadurch gekennzeichnet, daß sie mit einer speziellen Nukleotidsequenz hybridisiert, die wenigstens eine der folgenden prokaryontischen Sequenzen oder eine zu wenigstens einer der nachfolgenden Sequenzen komplementäre Sequenz beinhaltet: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA und TGGATCACCTCCTT, wobei das Verfahren eine in herkömmlicher Weise erfolgende Synthetisierung einer für die vorstehend genannte Sonde charakteristischen Nukleotidsequenz beinhaltet.

17. Verfahren zum Bilden eines Oligonukleotids, das eine Nukleotidsequenz enthalt, die ausgewählt ist aus der Gruppe bestehend aus: AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, TGGATCACCTCCTT, sowie dazu komplementären Sequenzen, wobei T Thymin, G Guanin, A Adenin, C Cytosin und - eine Nukleotidauslöschung innerhalb der Sequenz bedeuten,wobei das Verfahren eine in herktömmlicher Weise erfolgende Synthetisierung einer für das vorstehend genannte Oligonukleotid charakteristischen Nukleotidsequenz beinhaltet.

18. Verfahren zum Bilden eines Deoxyoligonukleotids, das die Nukleotidsequenz GCTTAGTCGATACAGC enthält, wobei das Verfahren eine in herkömmlicher Weise erfolgende Synthetisierung einer für das vorstehend genannte Deoxyoligonukleotid charakteristischen Nukleotidsequenz beinhaltet.

19. Verfahren zum Bilden eines Oligonukleotids, das die Nukleotidsequenz CGAAUCAGCUAUGUCG enthält, wobei das Verfahren eine in herkömmlicher Weise erfolgende Syntnetisierung einer für das vorstehend genannte Olionukleotid charakteristischen Nukleotidsequenz beinhaltet.

## Revendications (Revendications pour l'(les) Etat(s) contractant(s) suivant(s): BE, CH, DE, FR, GB, IT, LI, LU, NL, SE)

1. Procédé pour L'identification d'une séquence de nucléotides particulière destinée à être utilisée comme sonde spécifique des mycoplasmes ou comme sonde spécifique des Mollicutes en général, qui comprend les étapes consistant à:
a. cloner l'opéron entier du RNA ribosomique ou des fragments de celui-ci, d'une espèce particulière de mycoplasme dans un bactériophage ou un vecteur plasmidique;
b. sonder les fragments du gène RNA ribosomique obtenus à l'aide d'un opéron RNA ribosomique procaryote n'appartenant pas aux mycoplasmes ou des fragments de celui-ci,
c. caractériser les fragments qui s'hybrident avec ledit opéron RNA ribosomique procaryote n'appartenant pas aux mycoplasmes;
d. identifier les fragments spécifiques des mycoplasmes par hybridation sélective;
e. sous-cloner les fragments spécifiques des mycoplasmes dans un vecteur de séquençage;
f. séquencer les fragments sous-clonés obtenus spécifiques des mycoplasmes;
g. répéter les étapes a, b, c, d, e et f avec d'autres membres de la classe des Mollicutes;
h. comparer les séquences obtenues au cours des étapes f et g; et
i. identifier une séquence commune à toutes les espèces de mycoplasmes mais différant de la séquence correspondante chez les procaryotes non apparentés aux mycoplasmes en vue de l'utiliser comme une sonde spécifique des mycoplasmes; ou identifier des séquences spécifiques d'une espèce donnée de mycoplasmes, d'uréaplasmes, d'acholéplasmes, ou de spiroplasmes en vue de les utiliser comme des sondes spécifiques pour des espèces distinctes de mycoplasmes, d'uréaplasmes, d'acholéplasmes et de spiroplasmes.

2. Procédé pour rechercher la présence d'un organisme procaryote de la classe des Mollicutes, contenant un acide nucléique comportant une séquence de nucléotides particulière présente dans les acides nucléiques desdits organismes procaryotes mais absente des acides nucléiques provenant d'organismes eucaryotes, qui comprend les étapes consistant à:
identifier la séquence de nucléotides particulière selon le procédé de la revendication 1;
mettre un milieu susceptible de contenir un acide nucléique ou un fragment d'acide nucléique provenant dudit organisme procaryote comportant ladite séquence particulière de nucléotides en contact avec un oligonucléotide comportant une séquence de nucléotides complémentaire à ladite séquence de nucléotides particulière;
ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide.

3. Procédé selon la revendication 1 ou 2 dans lequel l'organisme procaryote est une espèce appartenant au genre Mycoplasma et ladite séquence de nucléotides particulière est présente uniquement dans les acides nucléiques de procaryotes appartenant à des espèces du genre Mycoplasma.

4. Procédé selon la revendication 1 ou 2 dans lequel l'organisme procaryote est Mycoplasma capricolum et ladite séquence de nucléotides particulière est présente uniquement dans les acides nucléiques de procaryotes de l'espèce Mycoplasma capricolum.

5. Procédé selon la revendication 1 ou 2 dans lequel l'organisme procaryote est Mycoplasma espèce PG50 et ladite séquence de nucléotides particulière est présente uniquement dans les acides nucléiques de procaryotes appartenant à Mycoplasma espèce PG50.

6. Procédé selon la revendication 1 ou 2 dans lequel l'organisme procaryote est Mycoplasma pneumoniae et ladite séquence de nucléotides particulière est présente uniquement dans les acides nucléiques de procaryotes de l'espèce Mycoplasma pneumoniae.

7. Procédé selon la revendication 1 ou 2 dans lequel l'organisme procaryote est une espèce d'uréaplasme et ladite séquence de nucléotides particulière est présente uniquement dans les acides nucléiques de procaryotes appartenant à ladite espèce d'uréaplasme.

8. Procédé selon la revendication 1 ou 2 dans lequel l'organisme procaryote est une espèce de spiroplasme et ladite séquence de nucléotides particulière est présente uniquement dans les acides nucléiques de procaryotes appartenant à ladite espèce de spiroplasme.

9. Procédé selon la revendication 1 ou 2 dans lequel l'organisme procaryote est une espèce d'acholéplasme et ladite séquence de nucléotides particulière est présente uniquement dans les acides nucléiques de procaryotes appartenant à ladite espèce d'acholéplasme.

10. Procédé selon la revendication 1 ou 2 dans lequel ledit fragment d'acide nucléique comportant ladite séquence de nucléotides particulière est le gène RNA 16S.

11. Procédé selon la revendication 1 ou 2 dans lequel ledit fragment d'acide nucléique comportant ladite séquence de nucléotides particulière est le gène RNA 23S.

12. Procédé selon la revendication 1 ou 2 dans lequel ledit fragment d'acide nucléique comportant ladite séquence de nucléotides particulière est le gène RNA 5S.

13. Procédé pour rechercher la présence d'un organisme procaryote de la classe des Mollicutes qui contient un acide nucléique comportant une séquence de nucléotides particulière présente dans les acides nucléiques provenant desdits organismes procaryotes mais absente des acides nucléiques d'organismes eucaryotes, ce procédé comprenant les étapes consistant à:
mettre un milieu susceptible de contenir un acide nucléique ou un fragment d'acide nucléique provenant dudit organisme procaryote comportant ladite séquence de nucléotides particulière en contact avec un oligonucléotide comportant une séquence de nucléotides complémentaire à ladite séquence de nucléotides particulière, ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide;
procédé caractérisé en ce que ladite séquence de nucléotides particulière comprend au moins l'une des séquences suivantes spécifiques des mycoplasmes ou une séquence complémentaire à au moins l'une des séquences suivantes; AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT et TAGATATATG.

14. Procédé pour rechercher la présence d'un organisme procaryote comprenant un acide nucléique comportant une séquence de nucléotides particulière présente dans les acides nucléiques d'organismes procaryotes mais absente dans les acides nucléiques d'organismes eucaryotes, qui comprend les étapes consistant à:
mettre un milieu susceptible de contenir un acide nucléique ou un fragment d'acide nucléique provenant dudit organisme procaryote comportant ladite séquence de nucléotides particulière en contact avec un oligonucléotide comportant une séquence de nucléotides complémentaire à ladite séquence de nucléotides particulière, ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide;
procédé caractérisé en ce que ladite séquence de nucléotides particulière comprend au moins l'une des séquences procaryotes suivantes ou une séquence complémentaire à au moins l'une des séquences suivantes: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA et TGGATCACCTCCTT.

15. Sonde spécifique pour les mycoplasmes, caractérisée en ce qu'elle s'hybride avec une séquence de nucléotides particulière comprenant au moins l'une des séquences suivantes spécifiques des mycoplasmes ou une séquence complémentaire à au moins une des séquences suivantes: AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT et TAGATATATG.

16. Sonde spécifique des procaryotes, caractérisée en ce qu'elle s'hybride avec une séquence de nucléotides particulière comprenant au moins l'une des séquences procaryotes suivantes ou une séquence complémentaire à l'une au moins des séquences suivantes : ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA et TGGATCACCTCCTT.

17. Oligonucléotide comportant une séquence de nucléotides choisie parmi le groupe constitué de AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA, TGGATCACCTCCTT et les séquences complémentaires à celles-ci, dans lesquelles T représente thymine, G représente guanine, A représente adénine, C représente cytosine et - indique une délétion de nucléotide au sein de la séquence.

18. Désoxyoligonucléotide comportant la séquence de nucléotides GCTTAGTCGATACAGC.

19. Oligonucléotide comportant la séquence de nucléotides CGAAUCAGCUAUGUCG.

## Revendications (Revendications pour l'(les) Etat(s) contractant(s) suivant(s): AT)

1. Procédé pour l'identification d'une séquence de nucléotides particulière destinée à être utilisée comme sonde spécifique des mycoplasmes ou comme sonde spécifique des Mollicutes en général, qui comprend les étapes consistant à:
a. cloner l'opéron entier du RNA ribosomique ou des fragments de celui-ci, d'une espèce particulière de mycoplasme dans un bactériophage ou un vecteur plasmidique;
b. sonder les fragments du gène RNA ribosomique obtenus à l'aide d'un opéron RNA ribosomique procaryote n'appartenant pas aux mycoplasmes ou des fragments de celui-ci,
c. caractériser les fragments qui s'hybrident avec ledit opéron RNA ribosomique procaryote n'appartenant pas aux mycoplasmes;
d. identifier les fragments spécifiques des mycoplasmes par hybridation sélective;
e. sous-cloner les fragments spécifiques des mycoplasmes dans un vecteur de séquençage;
f. séquencer les fragments sous-clonés obtenus spécifiques des mycoplasmes;
g. répéter les étapes a, b, c, d, e et f avec d'autres membres de la classe des Mollicutes;
h. comparer les séquences obtenues au cours des étapes f et g; et
i. identifier une séquence commune à toutes les espèces de mycoplasmes mais différant de la séquence correspondante chez les procaryotes non apparentés aux mycoplasmes en vue de l'utiliser comme une sonde spécifique des mycoplasmes; ou identifier des séquences spécifiques d'une espèce donnée de mycoplasmes, d'uréaplasmes, d'acholéplasmes, ou de spiroplasmes en vue de les utiliser comme des sondes spécifiques pour des espèces distinctes de mycoplasmes, d'uréaplasmes, d'acholéplasmes et de spiroplasmes.

2. Procédé pour rechercher la présence d'un organisme procaryote de la classe des Mollicutes contenant un acide nucléique comportant une séquence de nucléotides particulière présente dans les acides nucléiques desdits organismes procaryotes mais absente des acides nucléiques provenant d'organismes eucaryotes, qui comprend les étapes consistant à:
identifier la séquence de nucléotides particulière selon le procédé de la revendication 1;
mettre un milieu susceptible de contenir un acide nucléique ou un fragment d'acide nucléique provenant dudit organisme procaryote comportant ladite séquence particulière de nucléotides en contact avec un oligonucléotide comportant une séquence de nucléotides complémentaire à ladite séquence de nucléotides particulière;
ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide.

3. Procédé selon la revendication 1 ou 2 dans lequel l'organisme procaryote est une espèce appartenant au genre Mycoplasma et ladite séquence de nucléotides particulière est présente uniquement dans les acides nucléiques de procaryotes appartenant à des espèces du genre Mycoplasma.

4. Procédé selon la revendication 1 ou 2 dans lequel l'organisme procaryote est Mycoplasma capricolum et ladite séquence de nucléotides particulière est présente uniquement dans les acides nucléiques de procaryotes de l'espèce Mycoplasma capricolum.

5. Procédé selon la revendication 1 ou 2 dans lequel l'organisme procaryote est Mycoplasma espèce PG50 et ladite séquence de nucléotides particulière est présente uniquement dans les acides nucléiques de procaryotes appartenant à Mycoplasma espèce PG50.

6. Procédé selon la revendication 1 ou 2 dans lequel l'organisme procaryote est Mycoplasma pneumoniae et ladite séquence de nucléotides particulière est présente uniquement dans les acides nucléiques de procaryotes de l'espèce Mycoplasma pneumoniae.

7. Procédé selon la revendication 1 ou 2 dans lequel l'organisme procaryote est une espèce d'uréaplasme et ladite séquence de nucléotides particulière est présente uniquement dans les acides nucléiques de procaryotes appartenant à ladite espèce d'uréaplasme.

8. Procédé selon la revendication 1 ou 2 dans lequel l'organisme procaryote est une espèce de spiroplasme et ladite séquence de nucléotides particulière est présente uniquement dans les acides nucléiques de procaryotes appartenant à ladite espèce de spiroplasme.

9. Procédé selon la revendication 1 ou 2 dans lequel l'organisme procaryote est une espèce d'acholéplasme et ladite séquence de nucléotides particulière est présente uniquement dans les acides nucléiques de procaryotes appartenant à ladite espèce d'acholéplasme.

10. Procédé selon la revendication 1 ou 2 dans lequel ledit fragment d'acide nucléique comportant ladite séquence de nucléotides particulière est le gène RNA 16S.

11. Procédé selon la revendication 1 ou 2 dans lequel ledit fragment d'acide nucléique comportant ladite séquence de nucléotides particulière est le gène RNA 23S.

12. Procédé selon la revendication 1 ou 2 dans lequel ledit fragment d'acide nucléique comportant ladite séquence de nucléotides particulière est le gène RNA 5S.

13. Procédé pour rechercher la présence d'un organisme procaryote de la classe des Mollicutes qui contient un acide nucléique comportant une séquence de nucléotides particulière présente dans les acides nucléiques provenant desdits organismes procaryotes mais absente des acides nucléiques d'organismes eucaryotes, ce procédé comprenant les étapes comprenant les étapes consistant à:
mettre un milieu susceptible de contenir un acide nucléique ou un fragment d'acide nucléique provenant dudit organisme procaryote comportant ladite séquence de nucléotides particulière en contact avec un oligonucléotide comportant une séquence de nucléotides complémentaire à ladite séquence de nucléotides particulière, ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide;
procédé caractérisé en ce que ladite séquence de nucléotides particulière comprend au moins l'une des séquences suivantes spécifiques des mycoplasmes ou une séquence complémentaire à au moins l'une des séquences suivantes : AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT et TAGATATATG.

14. Procédé pour rechercher la présence d'un organisme procaryote comprenant un acide nucléique comportant une séquence de nucléotides particulière présente dans les acides nucléiques d'organismes procaryotes mais absente dans les acides nucléiques d'organismes eucaryotes, qui comprend les étapes consistant à:
mettre un milieu susceptible de contenir un acide nucléique ou un fragment d'acide nucléique provenant dudit organisme procaryote comportant ladite séquence de nucléotides particulière en contact avec un oligonucléotide comportant une séquence de nucléotides complémentaire à ladite séquence de nucléotides particulière, ce par quoi ledit oligonucléotide s'hybride avec tout acide nucléique ou fragment d'acide nucléique provenant dudit organisme procaryote comportant ladite séquence de nucléotides particulière susceptible d'être présent dans ledit milieu; et
détecter la présence de tout acide nucléique ou fragment d'acide nucléique hybridé audit oligonucléotide;
procédé caractérisé en ce que ladite séquence de nucléotides particulière comprend au moins l'une des séquences procaryotes suivantes ou une séquence complémentaire à au moins l'une des séquences suivantes: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA et TGGATCACCTCCTT.

15. Procédé pour préparer une sonde spécifique pour les mycoplasmes, caractérisée en ce qu'elle s'hybride avec une séquence de nucléotides particulière comprenant au moins l'une des séquences suivantes spécifiques des mycoplasmes ou une séquence complémentaire à au moins une des séquences suivantes : AACACGTATC, CGAATCAGCTATGTCG, GAGGTT-AAC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT et TAGATATATG, lequel procédé comprend la synthèse de manière conventionnelle d'une séquence de nucléotides caractéristique de la sonde précitée.

16. Procédé pour préparer une sonde spécifique des procaryotes, caractérisée en ce qu'elle s'hybride avec une séquence de nucléotides particulière comprenant au moins l'une des séquences procaryotes suivantes ou une séquence complémentaire à au moins l'une des séquences suivantes: ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCTACAATG, CTAGTAATCG, TGTACACACCGCCCGTCA, AAGTCGTAACAAGGTA et TGGATCACCTCCTT, lequel procédé comprend la synthèse de manière conventionnelle d'une séquence de nucléotides caractéristique de la sonde précitée.

17. Procédé pour préparer un oligonucléotide comportant une séquence de nucléotides choisie parmi le groupe constitué de AACACGTATC, CGAATCAGCTATGTCG, GAGGTT-AACC, ATCCGGATTTATT, TCTCAGTTCGGATTGA, AGGTGGTGCATGGTTG, TCCTGGCTCAGGAT, ATACATAGGT, AACTATGTGC, AATTTTTCACAATG, TCTCGGGTCT, TAGATATATG, ACGGGTGAGT, TAATACCGCAT, TACGGGAGGCAGCAGT, GTGGGGAGCAAA, AGGATTAGATACCCT, CCGTAAACGAT, GAATTGACGGGG, CCCGCACAAG, GGTGGAGCATGT, TGTTGGGTTAAGTCCCGCAACGA, GGGATGACGT, ACGTGCCTACAATG, CTAGTAATCG, TGTACACACCGCCCGTCA, AAGTCGTAACAAGGTA, TGGATCACCTCCTT et les séquences complémentaires à celles-ci, dans lesquelles T représente thymine, G représente guanine, A représente adénine, C représente cytosine et - indique une délétion de nucléotide au sein de la séquence, lequel procédé comprend la synthèse de manière conventionnelle d'une séquence de nucléotides caractéristique de l'oligonucléotide précité.

18. Procédé pour la préparation d'un désoxyoligonucléotide comportant la séquence de nucléotides GCTTAGTCGATACAGC, lequel procédé comprend la synthèse de manière conventionnelle d'une séquence de nucléotides caractéristique du désoxyoligonucléotide précité.

19. Procédé pour la préparation d'un oligonucléotide comportant la séquence de nucléotides CGAAUCAGCUAUGUCG, lequel procédé comprend la synthèse de manière conventionnelle d'une séquence de nucléotides caractéristique de l'oligonucléotide précité.
